# EUROPÄISCHE PATENTSCHRIFT

(11) **EP 0 854 856 B1**
(45) Veröffentlichungstag und Bekanntmachung des Hinweises auf die Patenterteilung: **05.04.2000**
(21) Anmeldenummer: 96927664.1
(22) Anmeldetag: 02.08.1996
(51) Int. Cl.: C07C 209/36, C07C 213/02

(54) **VERFAHREN ZUR REDUKTION VON NITROVERBINDUNGEN**
NITRO COMPOUND-REDUCING PROCESS
PROCEDE PERMETTANT DE REDUIRE DES COMPOSES NITRO

(30) Priorität: 14.08.1995 DE 19529874; 14.05.1996 DE 19619359
(43) Veröffentlichungstag der Anmeldung: 29.07.1998
(73) Patentinhaber: Clariant GmbH, 65929 Frankfurt am Main (DE)
(72) Erfinder: BAHRMANN, Helmut, D-46499 Hamminkeln (DE); CORNILS, Boy, D-65719 Hofheim (DE); DIERDORF, Andreas, D-60529 Frankfurt am Main (DE); HABER, Steffen, D-76726 Germersheim (DE)
(86) Internationale Anmeldenummer: EP9603415
(87) Internationale Veröffentlichungsnummer: WO9707087

(56) Entgegenhaltungen:
- EP-A- 0 372 313
- JOURNAL OF ORGANIC CHEMISTRY, Bd. 41, Nr. 7, 2.April 1976, EASTON US, Seiten 1200-1205, XP002018366 J. F. KNIFTON: "Homogenous Catalyzed Reduction of Nitro Compounds. IV. Selective and Sequential Hydrogenation of Nitroaromatics" in der Anmeldung erwähnt
- TETRAHEDRON LETTERS, Bd. 36, Nr. 51, 18.Dezember 1995, OXFORD GB, Seiten 9305-9308, XP002018367 AHMED M. TAFESH ET AL.: "First Selective Reduction of Aromatic Nitro Compounds Using Water Soluble Catalysts" in der Anmeldung erwähnt

## Beschreibung

Die Erfindung betrifft ein Verfahren zur Reduktion von Nitroverbindungen zu den entsprechenden Aminen unter Verwendung eines homogenen Palladium, Ruthenium, Nickel oder Rhodiumkatalysators.

Die Reduktion von Nitroverbindungen, speziell von Nitroaromaten zu den entsprechenden Aminen bzw. Anilinen wird schon seit über hundert Jahren industriell durchgeführt. Während bis vor wenigen Jahren noch die Reduktion mittels Eisen großtechnisch angewendet wurde, hat in neuerer zeit die katalytische Reduktion mit Edelmetall-Katalysatoren auf verschiedenen Trägermaterialien in der Industrie Einzug gehalten. Obwohl diese Methode viele Vorteile gegenüber der Eisenreduktion hat, ergeben sich in der Praxis doch häufig Probleme. So sind häufig lange Filtrationszeiten beim Abtrennen des Katalysators nötig. Die Katalysatoren sind meist äußerst empfindlich gegen Spuren von Katalysatorgiften wie Schwefelverbindungen niederer Oxidationsstufen, was zum völligen Stillstand der Reduktion führen kann. Die Fehlersuche bei einem Mißlingen der Reaktion ist aufgrund der Natur eines heterogenen Katalysators oft problematisch und teilweise nur mit erheblichem apparativem Aufwand möglich.

Enthält der Nitroaromat Halogensubstituenten kommt es häufig zur Dehalogierung als Nebenreaktion. Diese kann durch selektive Deaktivierung des Katalysators unterdrückt werden, wobei aber die Reproduzierbarkeit erschwert ist.

Reduktionen mit homogenen Katalysatoren, die gegenüber heterogenen Katalysatoren häufig effizienter sind, werden beispielsweise in J. Org. Chem. 1976, 41, 1200 und J. Indian Chem. Soc. 1986, 63, 901 beschrieben.

Aufgrund der Probleme beim Abtrennen des Katalysators vom Produkt sind diese Reaktionen bislang nur im Labormaßstab durchgeführt worden. Auch hier tritt bei halogenierten Nitroaromaten Dehalogenierung als Nebenreaktion auf.

Aufgabe was es daher ein Verfahren bereitzustellen, welches die genannten Nachteile zumindest vermindert und die Reduktion von Nitroverbindungen zu Aminen in einfacher Weise und in hohen Ausbeuten ermöglicht.

Es wurde nun gefunden, daß sich die Reduktion von Nitroverbindungen mit Wasserstoff in einem wäßrigen System unter Edelmetallkatalyse schonend und in hohen Ausbeuten durchführen läßt, wenn die Reaktion in Anwesenheit eines wasserlöslichen Liganden durchgeführt wird.

In der EP-A-0 372 313 ist die Verwendung von Edelmetallkatalysatoren mit Tris-(m-sulfophenyl)-phosphan als Komplexliganden beschrieben. Diese Katalysatoren werden insbesondere bei der Wassergas-Reaktion, bei Hydrocarbonylierungen und Hydroformulierungen eingesetzt.

Es war daher überraschend, daß die genannten Katalysatoren auch für die Reduktion von Nitroaromaten zu den entsprechenden Anilinen geeignet sind, ohne daß beispielsweise eine Dehalogenierung oder C'/C-Verknüpfung unter Dehalogenierung zu beobachten ist.

Mit wasserlöslichen Katalysatoren konnten Nitroaromaten bisher nur unter Verwendung von Kohlenmonoxid oder Ameisensäurederivaten zu Anilinen reduziert werden (Tetrahedron Letters 1995, 36, 9305). Für die technische Realisierung dieser Verfahren ist die akute Toxizität von Kohlenmonoxid und der relativ hohe Preis der Ameisensäurederivate von Nachteil.

Gegenstand der Erfindung ist daher ein Verfahren zur Reduktion von Nitrogruppen zu Aminogruppen, dadurch gekennzeichnet, daß man eine Nitroverbindung mit Wasserstoff in Gegenwart eines wasserlöslichen Metallkatalysators der allgemeinen Formel (I) umsetzt,

M(L)ₙ(Y)ₘ (I)

wobei die Symbole und Indizes folgende Bedeutungen haben:
- M: ist Ruthenium, Rhodium, Nickel oder Palladium;
- L: ist gleich oder verschieden ein wasserlöslicher Ligand;
- Y: ist gleich oder verschieden ein weiterer Ligand oder ein Alkali- oder Erdalkaliion;
- n: ist 1, 2, 3 oder 4;
- m: ist 0, 1 oder 2.

Mit dem erfindungsgemäßen Verfahren werden Nitroverbindungen in sehr hoher Ausbeute und Selektivität reduziert, wobei es im Falle von halogenierten Nitroverbindungen nur wenig zu Dehalogenierungsreaktionen kommt. Die Abtrennung der wäßrigen Katalysatorlösung kann durch einfache Phasentrennung erfolgen.

M ist vorzugsweise Rhodium, Ruthenium oder Palladium.

Als Reaktionsmedium dient vorzugsweise Wasser, der wasserlösliche Katalysator wird vorzugsweise in Form einer wäßrigen Lösung eingesetzt.

Für das erfindungsgemäße Verfahren geeignete wasserlösliche Liganden enthalten beispielsweise Sulfonsäuresalz- und/oder Sulfonsäurereste und/oder Carbonsäuresalz- und/oder Carbonsäurereste und/oder Phosphonsäuresalz- und/oder Phosphonsäurereste und/oder Phosphinsäuresalz- und/oder Phosphinsäurereste, und/oder Phosphoniumgruppen und/oder Peralkylammoniumgruppen und/oder Hydroxygruppen und/oder Polyethergruppen mit geeigneter Kettenlänge, vorzugsweise mit mehr als 10 Alkylenoxideinheiten. Bevorzugt sind Sulfonsäuresalz-, Sulfonsäure-, Carbonsäuresalz-, Carbonsäure-, Phosphonsäuresalz- und Phosphonsäuregruppen.

Bevorzugte Klassen von wasserlöslichen Liganden sind mit den obigen Gruppen substituierte Phosphane, wie Trialkylphosphane, Tricycloalkylphosphane, Triarylphosphane, Dialkylarylphosphane, Alkyldiarylphosphane und Heteroarylphosphane, wie Tripyridylphosphan und Trifurylphosphan, wobei die drei Substituenten am Phosphor gleich oder verschieden, chiral oder achiral sein können und wobei einer oder mehrere der Substituenten die Phosphorgruppen mehrerer Phosphane verknüpfen können und wobei ein Teil dieser Verknüpfung auch ein oder mehrere Metallatome sein können, Phosphite, Phosphinigsäureester und Phosphonigsäureester, Phosphole und Dibenzophosphole. wozu auch Phosphoratome enthaltende cyclische bzw. oligo- und polycyclische Verbindungen zählen.

Weitere geeignete Gruppen wasserlöslicher Komplexliganden umfassen beispielsweise Bipyridine, Phenanthroline, Porphyrine und Alizarine, die mit den obengenannten Gruppen modifiziert sind.

Bevorzugt eingesetzte wasserlösliche Phosphane sind wasserlösliche Triarylphosphane, die neben den die Wasserlöslichkeit bewirkenden Substituenten, wie F, Alkyl- oder Alkoxygruppen mit vorzugsweise 1 - 12 Kohlenstoffatomen, enthalten können, sowie solche der allgemeinen Formeln (II) bis (VIII), 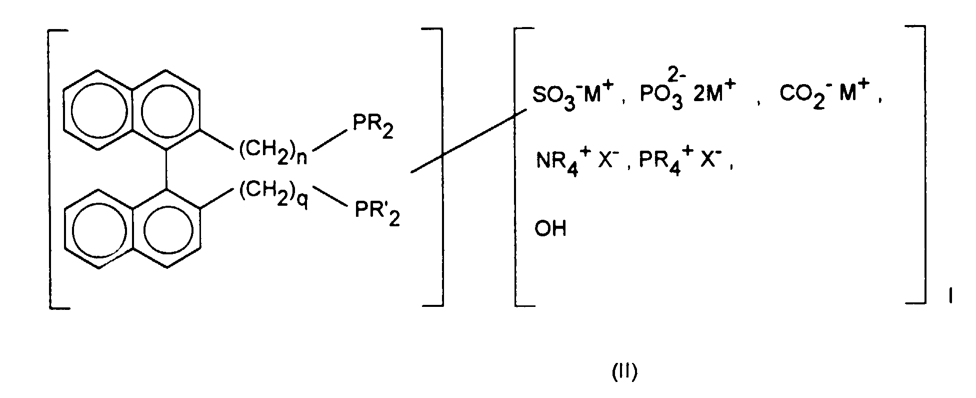 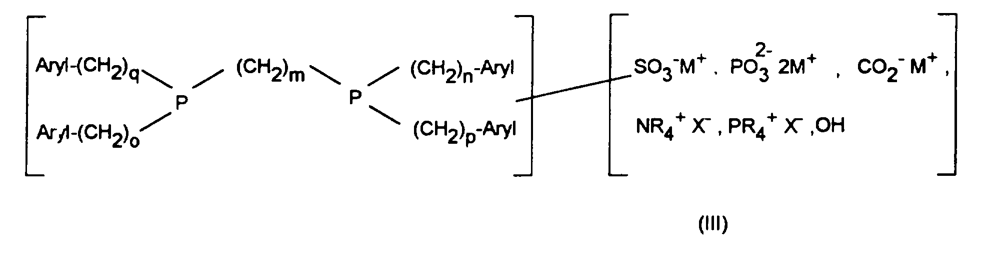 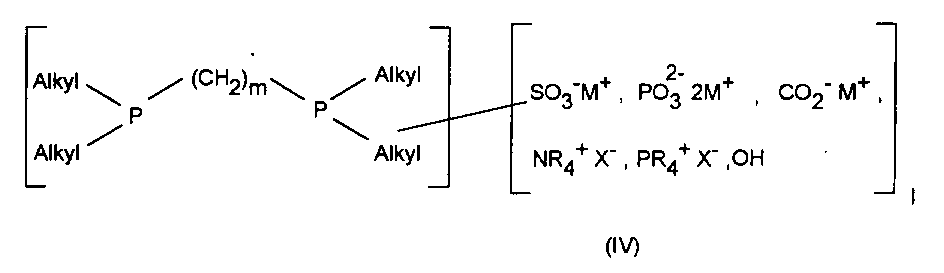 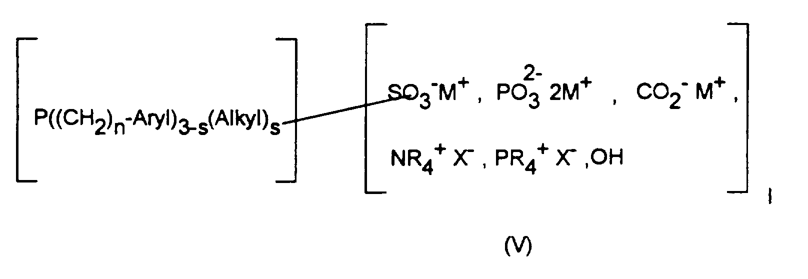 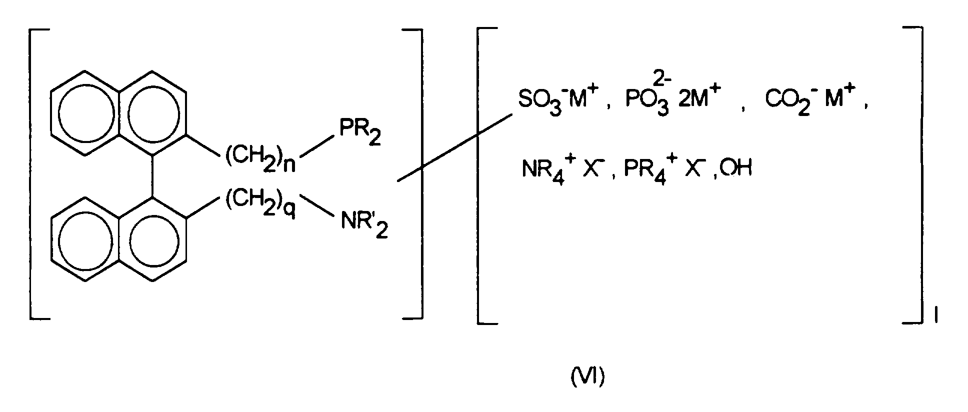 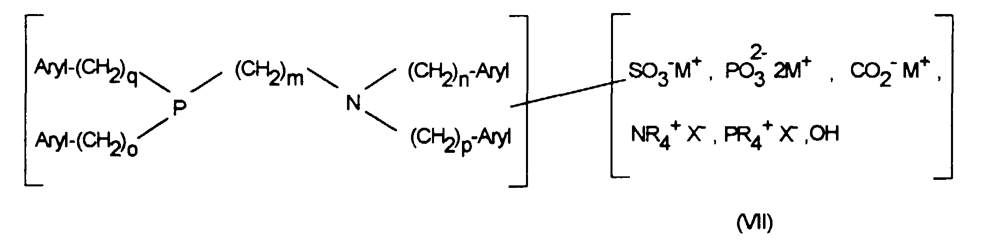 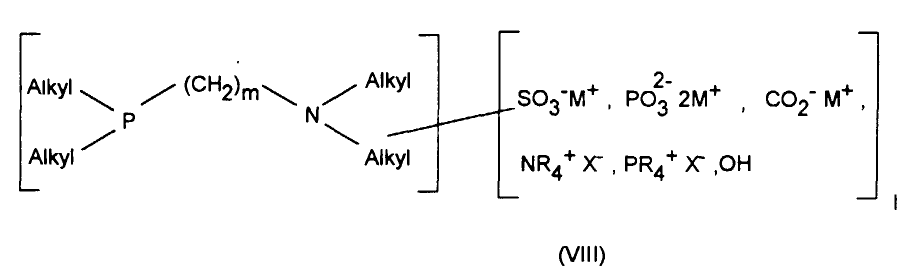 wobei die Symbole und Indizes folgende Bedeutungen haben:
- Aryl:: eine Phenyl- oder Naphthylgruppe, die auch einen oder mehrere Substituenten R tragen kann;
- Alkyl:: : eine geradkettige oder verzweigte Alkylgruppe mit 1 bis 8 Kohlenstoffatomen;
- R,R':: F oder vorzugsweise Alkyl, Aryl oder Aralkyl mit 1 bis 18 Kohlenstoffatomen;
- M:: Alkali-, Erdalkalimetall oder NR₄;
- X:: Halogen, BF₄, PF₆, OSO₂CF₃, 1/2[S0₄];
- l,m:: 1 bis 8;
- n,o,p,q:: 0, 1 bis 8;
- s:: 0, 1 bis 3.

Im folgenden sind Beispiele für besonders bevorzugte wasserlösliche Komplexliganden aufgeführt:
(R hat dabei, wenn nicht anders vermerkt, die in den Formeln (II) bis (VIII) angegebenen Bedeutungen)
1. Sulfonierte Phosphane 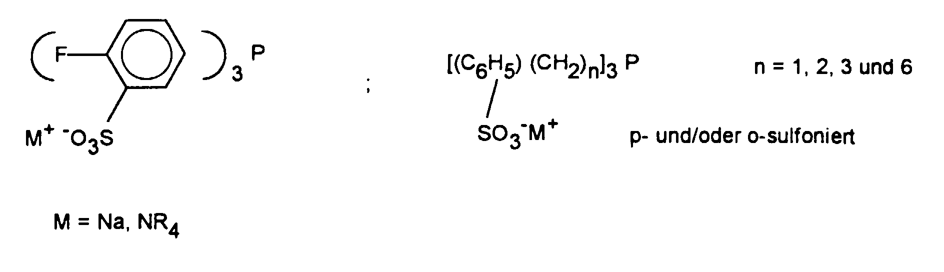 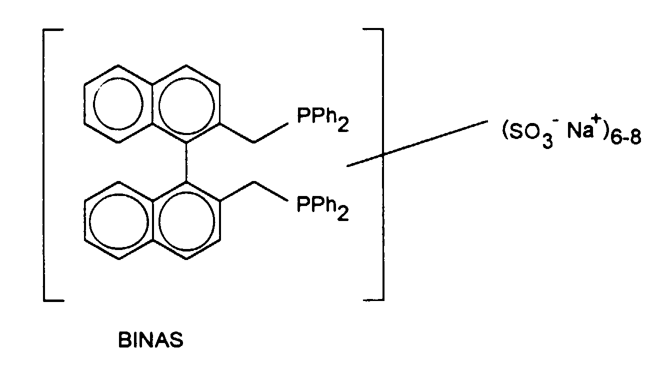 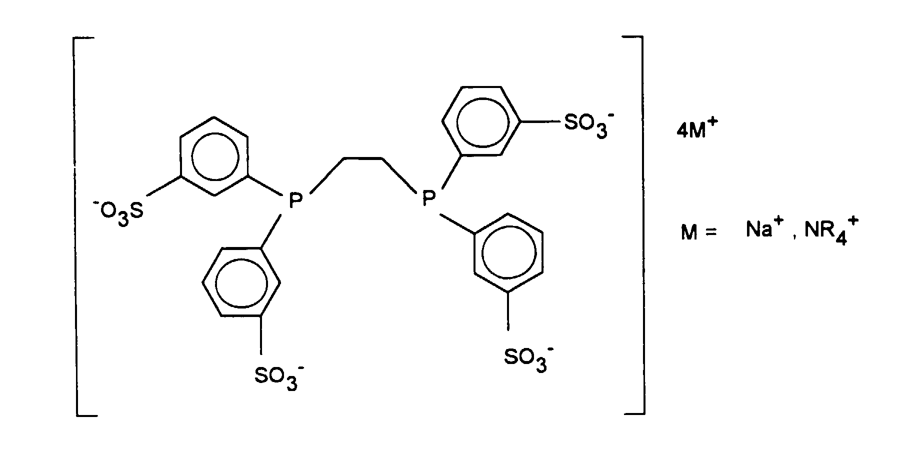 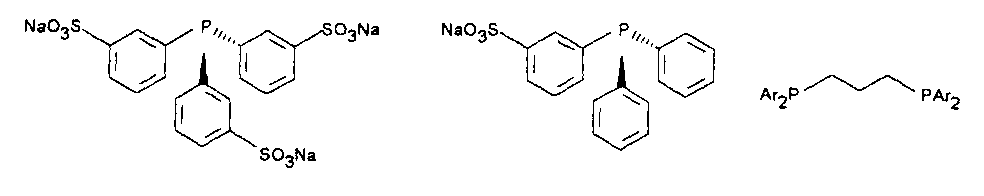 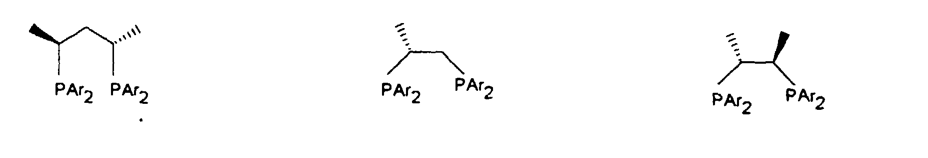 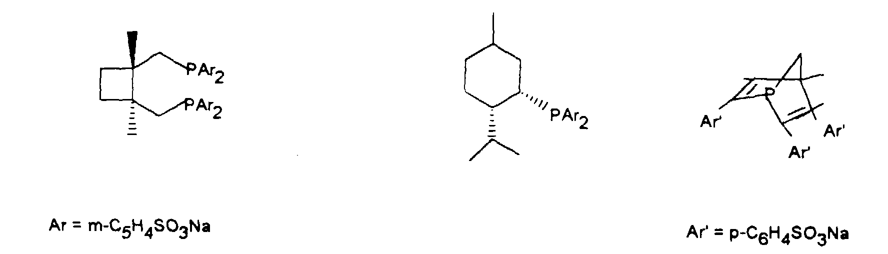 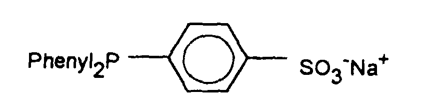 R₃₋ₙP(p-C₆H₄SO₃K)ₙ R = C₆H₅, 2-Pyridyl, 3-Pyridyl; n = 1-3 P[p-OC₆H₄SO₃(NH(i-octyl)₃]₃
2. Phosphane mit hydrophilen Gruppen in der Peripherie 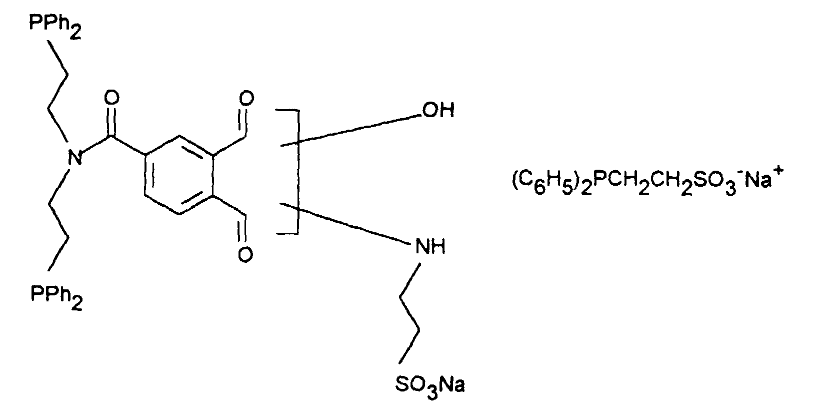 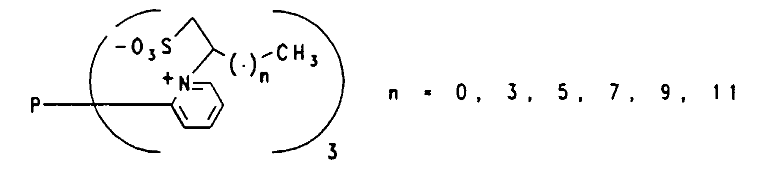
3. Phosphane mit quaternisierten Aminoalkyl- und Aminoaryl-Substituenten 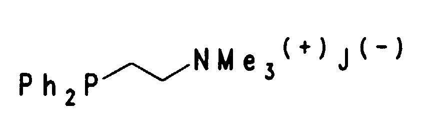 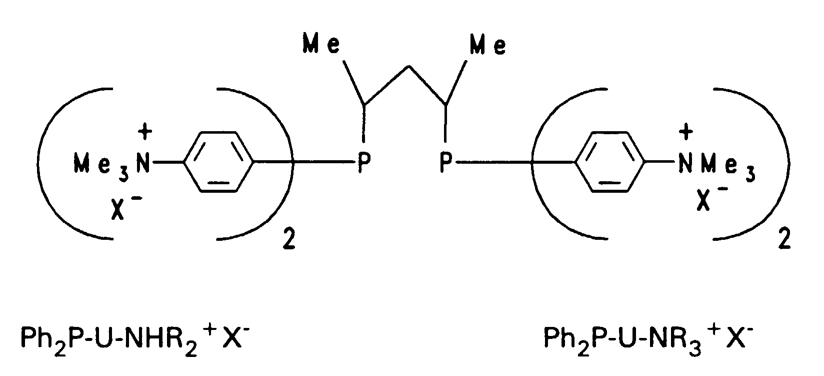 U = -CH₂CH₂-, -CH(CH₃)CH₂-, -CH₂CH(CH₃)CH₂-; R = CH₃; X = l^{⊖} Br^{⊖}, Cl^{⊖}, OSO₂CF₃^{⊖} , BF₄^{⊖}, PF₆^{⊖}
4. Carboxylierte Phosphane 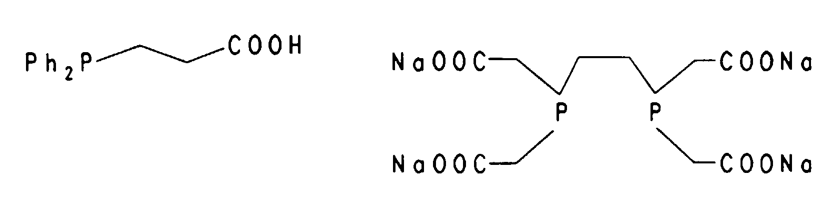 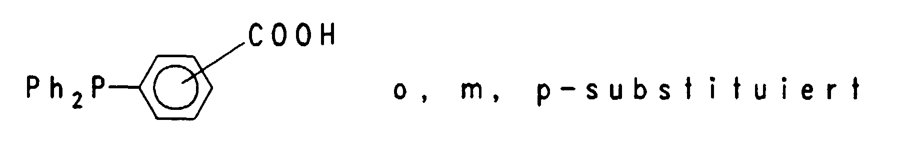 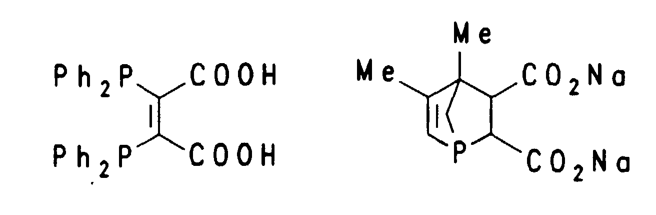
5. Phosphane mit Hydroxyalkyl- oder Polyether-Substituenten 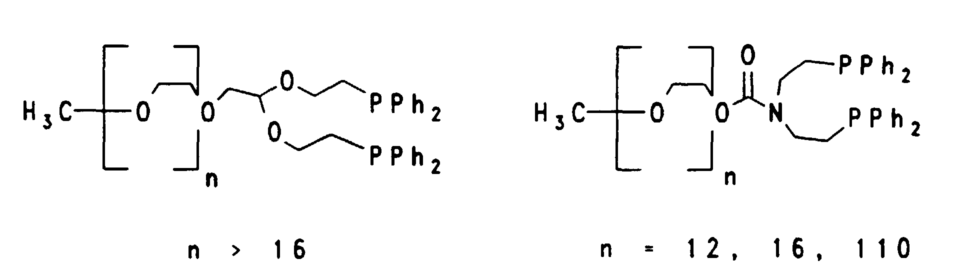 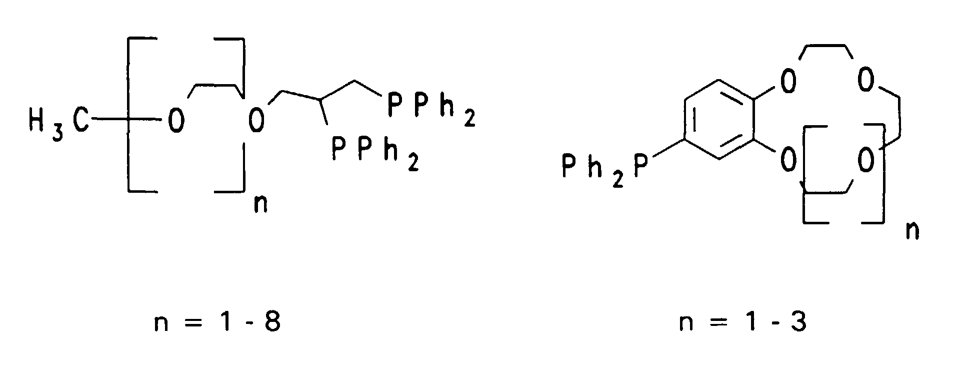
6. Phosphinoalkyl-phosphoniumsalze 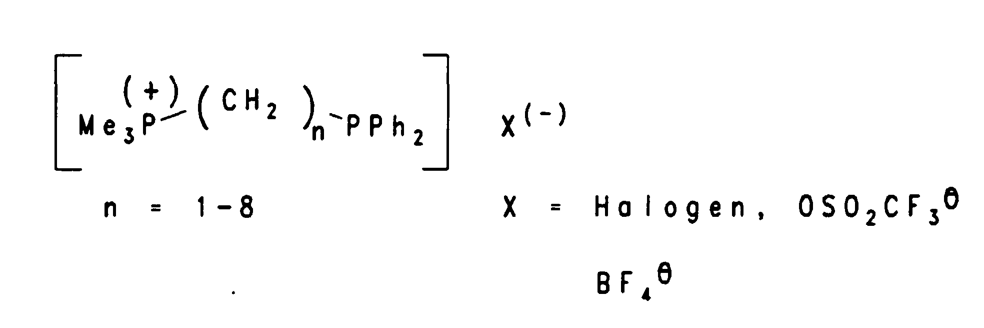
7. Phosphite

   P[-OC₆H₄SO₃{NH(i-Octyl)₃}]₃

   Insbesondere bevorzugte wasserlösliche Phosphanliganden sind:

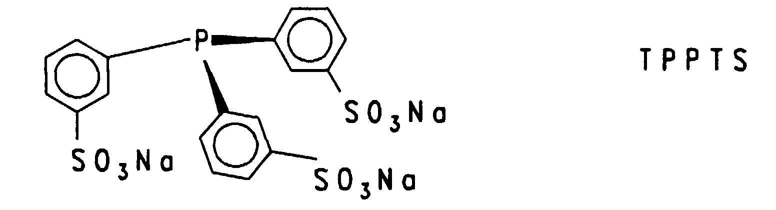

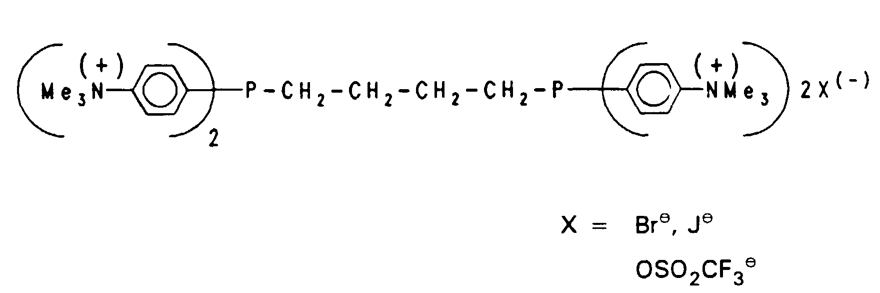

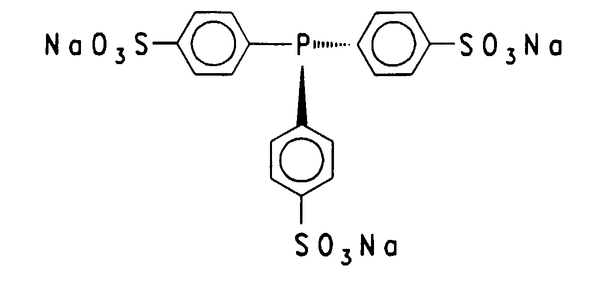

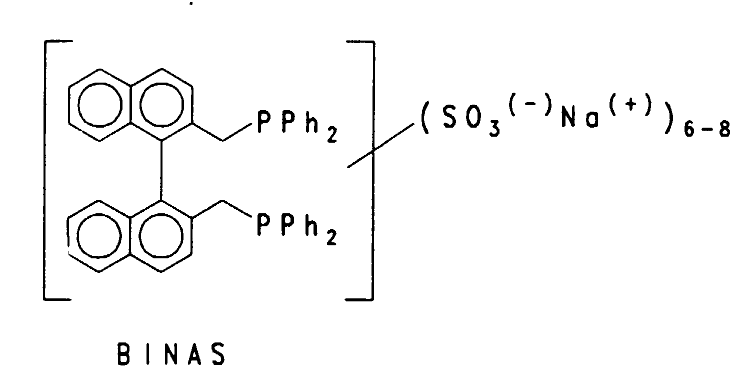

In der Formel (I) ist
- Y: ist vorzugsweise H, CO, CN oder Halogen, vorzugsweise Cl, oder ein Alkali- oder Erdalkaliion, vorzugsweise Na, K oder Li.

Falls M Palladium ist, bedeutet Y besonders bevorzugt Cl^{⊖}, CO, Na, K, Li. Falls M Rhodium ist, bedeutet Y besonders bevorzugt Cl^{⊖}, CO, Na, K, Li, H.

Die erfindungsgemäß eingesetzten wasserlöslichen Komplexliganden sind zum großen Teil aus der Literatur bekannt. Die Synthesen dieser Verbindungen sind beispielsweise beschrieben in W.A. Herrmann und C.W. Kohlpainter, Angew. Chem. Int. Ed. Engl. 32 (1993) 1524 und der dort zitierten Literatur oder können nach literaturbekannten oder analogen dem Fachmann geläufigen Methoden erfolgen. Die Herstellung von BINAS ist in der EP-A 0 571 819 bzw. US-A 5,347,9045 beschrieben.

Es können gegebenenfalls auch Mischungen zweier oder mehrerer verschiedener wasserlöslicher Liganden eingesetzt werden.

Der wasserlösliche Metallkatalysator der Formel (I) kann separat synthetisiert werden oder aber in situ durch Zusammengabe eines Ruthenium-, Nickel-, Palladium- oder Rhodiumsalzes, wie Rhodiumchloridhydrat oder Rutheniumchlorid, wie Rhodiumchlorid-hydrat, Rutheniumchlorid, Nickelhalogenid bzw. Palladium(II)salze und eines wasserlöslichen Liganden hergestellt werden.

Gegebenenfalls wird ein Reduktionsmittel zugesetzt. In der Regel ist Wasserstoff das geeignete Reduktionsmittel zur Darstellung der aktiven Katalysatorspezies.

Die erfindungsgemäß eingesetzten Metallkomplexe der Formel (I) sind teilweise aus der Literatur bekannt (siehe z.B. EP-A-0 372 313 oder J. Organomet. Chem. 1990, 389, 103).

Besonders bevorzugte Metallkomplexe der Formel (I) sind Rh(CO)Cl(TPPTS)₂, wobei TPPTS Triphenylphosphanbisulfonat-tri-natriumsalz bedeutet, Pd(TPPTS)₂ und RU(TPPTS)₂Cl₂.

Gegenstand der Erfindung ist weiterhin ein Verfahren zur Herstellung von Rh(CO)Cl(TPPTS)₂. Der genannte Katalysator kann nach einem neuen Verfahren leicht aus einer wäßrigen Lösung von TPPTS und Rhodiumtrichlorid sowie nachträglicher Zugabe von Formaldehyd mit anschließendem Erhitzen gewonnen werden, wie im folgenden im Beispiel 1 beschrieben ist. Dieses Verfahren ist gegenüber dem in EP-0 372 313 beschriebenen wesentlich einfacher. Eine Isolierung und chromatographische Reinigung der Zwischenstufen entfällt. Das TPPTS kann als technische Ware, so wie es in der Produktion anfällt, eingesetzt werden. Das Kohlenmonoxid wird durch die wäßrige Formaldehyd-Lösung ersetzt, so daß die apparative Handhabung auch hier wesentlich vereinfacht wird.

Das erfindungsgemäße Verfahren wird zweckmäßigerweise in einem Autoklaven durchgeführt.
Autoklaven sind - nach Römpps Chemie Lexikon - verschließbare, auf hohen Überdruck geprüfte Metallgefäße mit aufgeschraubtem, durch Bajonettverschluß gesichertem oder aufgepreßtem, dicht schließendem Deckel. In diesem Autoklaven-Kopf befinden sich beispielsweise Anschlußmöglichkeiten für eine Berstplatte oder ein Sicherheitsventil, Manometer, Thermometer und oft auch für einen gasdicht angebrachten Rührapparat. Neben stationären Autoklaven mit Innenrührung (auch Magnetrührung) gibt es auch Schüttel-Autoklaven und rotierende Autoklaven. Die Beheizung des Autoklaven erfolgt bei Labor-Autoklaven (Fassungsvermögen von ca. 100 ml bis zu mehreren Litern) meist elektrisch, beigrößeren Autoklaven in der industriellen Verfahrenstechnik (bis zu mehreren m³) meist durch Dampf. Autoklaven, in denen Reaktionen unter Verbrauch von Gasen ablaufen, z.B. Druckhydrierung, enthalten zusätzlich einen Einlaß für das gasführende Ausgangsmaterial, das meist von einem Kompressor gefördert wird. In der Mehrzahl der Fälle besteht das Material der Autoklaven aus hochlegiertem Stahl (z.B. V4A), doch gibt es auch spezielle Autoklaven aus Kupfer, Leicht- und Monelmetall.

Der Katalysator, enthaltend einen Metallkomplex der Formel (I), wird vorzugsweise in wäßriger Lösung eingesetzt, wobei die Menge an Wasser in einem großen Bereich variieren kann. Da die Reaktion in der Wasserphase stattfindet, wird sich durch die Bereitstellung größerer Mengen Wasser mehr Nitroaromat im Wasser lösen, wodurch die Reduktion beschleunigt wird. Die Wassermenge hat somit in gewissen Grenzen einen Einfluß auf die Reaktionsgeschwindigkeit. Da der Katalysator selbst extrem gut in Wasser löslich ist, ist man auch hier in der Wassermenge nicht eingeschränkt. Außerdem ist zu berücksichtigen, daß sich bei der Reduktion selbst Wasser bildet. Bei einem Mol Substrat und einem Millimol Katalysator setzt man im allgemeinen 10 - 1 000 ml, bevorzugt 50 - 500 ml, insbesondere 100 - 200 ml Wasser ein. Das molare Verhältnis Katalysator zu Substrat beträgt im allgemeinen 1:100 bis 1:100 000, bevorzugt 1:500 bis 1:10 000, insbesondere 1:500 bis 1:5 000. Aus den Verhältnissen Katalysator zu Substrat und Katalysator zu Wasser ergibt sich ein Verhältnis von Katalysator zu Wasser von ungefähr 1:500 bis 1:200 000, bevorzugt 1:2 000 bis 1:190 000, insbesondere 1:5 000 bis 1:100 000.
Wird als wasserlöslicher Katalysator Rh(CO)CL(TPPTS)₂ verwendet, beträgt das Verhältnis von Substrat zu Katalysator insbesondere 1:2000 bis 1:5000.

Die jeweilige Reaktionsgeschwindigkeit hängt von vielen Parametern ab, wie Druck, Temperatur, der Menge an Katalysator, der Menge an Substrat und der Menge an Wasser. Sie hängt aber auch entscheidend von der Löslichkeit des Substrats in Wasser ab. Nitroaromaten, die z.B. eine Ethoxy- oder Methoxygruppe enthalten, sind in der Regel besser wasserlöslich als alkylierte Verbindungen. Die Reaktionszeit beträgt üblicherweise je nach den Reaktionsbedingungen und der Natur des Nitroaromaten 30 min bis 10 h. Die Wasserstoffaufnahme bricht in der Regel nach vollständiger Reduktion scharf ab.

Die Reaktionstemperatur liegt im allgemeinen bei 20 bis 150°C, bevorzugt bei 50 - 130°C, insbesondere bei 60 bis 110°C. der Wasserstoffdruck kann 1 - 150 bar, bevorzugt 10 - 100 bar, besonders bevorzugt 10 - 20 bar betragen. Häufig gewählte Reaktionsbedingungen sind 100°C und 20 bar Wasserstoffdruck. Wird als wasserlöslicher Katalysator Rh(CO)Cl(TPPTS)₂ verwendet, liegt die bevorzugte Reaktionstemperatur bei 70 bis 130°C, insbesondere bei 90 bis 110°C.

Das Reaktionsprodukt wird nach Beendigung der Reduktion von der wäßrigen Katalysatorlösung abgetrennt. Die wäßrige Katalysatorlösung kann für weitere Reduktionen verwendet werden. Ein Aktivitätsverlust wird bei der Rückführung nicht beobachtet. Bei mehrmaliger Rückführung des Katalysators wird die wäßrige Katalysatorlösung durch das hinzukommende Reaktionswasser immer stärker verdünnt. Beispielsweise durch Membranfilter oder durch Einengen im Vakuum können die ursprünglichen Konzentrationsverhältnisse wiederhergestellt werden.

Das Katalysatorsystem ist relativ luftunempfindlich, dennoch sollte die wäßrige Lösung inert behandelt werden, um eine möglichst lange Lebensdauer und Rückführbarkeit des Katalysators zu gewährleisten.

Bei der Reduktion kann auf den Zusatz ein is organischen Lösungsmittels für den Nitroaromaten verzichtet werden. Damit entfällt auch die aufwendige und kostenintensive Lösungsmittelaufbereitung. Auch die bei der heterogenen katalytischen Reduktion notwendige Filtration vom Katalysator entfällt. Es ist jedoch auch möglich, daß man die Nitroverbindung mit einem organischen Lösungsmittel, beispielsweise mit Toluol, o-Xylol, m-Xylol, p-Xylol, Gemischen isomerer Xylole, Ethylbenzol, Mesitylen, Chlorbenzol, Dichlorbenzol, Chlortoluol, Cyclohexan, Cumol, Dekalin, Ethylacetat, Butylacetat oder einem Gemisch aus zwei oder mehreren dieser Lösungsmittel, verdünnt.

Bevorzugte Edukte des erfindungsgemäßen Verfahrens sind aromatische Nitroverbindungen. Besonders bevorzugte Ausgangsverbindungen sind solche der Formel (IX) 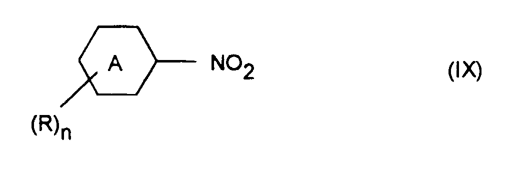 worin die Symbole und Indizes folgende Bedeutungen haben:
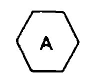 ist ein aromatischer Kohlenwasserstoff, der gegebenenfalls auch Heteroatome, vorzugsweise -O- und/oder -N- und/oder -S-, enthalten kann, mit 4 bis 20 Kohlenstoffatomen, vorzugsweise Phenyl oder Naphthyl;
n: ist je nach aromatischem Grundkörper 0, 1, 2, 3, 4 oder 0, 1, 2, 3, 4, 5, 6, vorzugsweise 0, 1 oder 2;
R: ist gleich oder verschieden -H, -F, -Cl, -Br, -l, CN, -SCN, -OCN, -OH, -NH₂, -CHO, -NO₂, -SO₃H, R', OR', -COOR', -OCOR', -NHR',-NR'₂, NHCOR';
R': ist gleich oder verschieden H, eine verzweigte oder unverzweigte Alkyl-, Alkenyl- oder Alkinylgruppe mit 1 bzw. 2 bis 12, vorzugsweise 1 bzw. 2 bis 8, Kohlenstoffatomen, bei der eine oder mehrere CH₂-Gruppen durch -O- und/oder ein oder mehrere H-Atome durch -F, -Cl, -Br, -l, -OH und/oder -NH₂ ersetzt sein können; oder ein aromatischer Kohlenwasserstoffrest, der gegebenenfalls auch Heteroatome, vorzugsweise -O-, -N- und/oder -S- enthalten kann und der gegebenenfalls auch weitere Substituenten R enthalten kann, mit 4 bis 20 Kohlenstoffatomen, vorzugsweise Phenyl oder Naphthyl.

Bevorzugt sind Verbindungen der Formel (IX) in denen
- n: 0, 1 oder 2 ist und
- R: eine Methyl-, Ethyl-, n-Propyl-, Isopropyl-, n-Butyl-, tert.-Butyl-, n-Pentyl-, i-Pentyl-, n-Hexyl-, n-Heptyl-, n-Octyl-, 2-Ethylhexyl-, 2-Ethyldecyl-, n-Decyl-, n-Dodecyl-, ferner eine Methoxy-, Ethoxy-, Propoxy-, Isopropoxy-, Butoxy-, Pentyloxy-, Hexyloxy-, Octyloxy-, Hydroxy, 2-Ethylhexyloxy-, Decyloxy-, Dodecyloxy-, ferner eine Carbonsäure-, Carbonsäuremethylester-, Carbonsäureethylester-, Carbonsäurepropylester-, Carbonsäurebutylester-, Carbonsäurepentylester-, Carbonsäurehexylester-, Carbonsäure-2-ethylhexylester-, Carbonsäurephenylester-, Formyl-, Carbonsäureamid-, Carbonsäuredimethylamid-, Carbonsäurediethylamid-, Carbonsäuremethylamid-, Carbonsäureethylamid-, ferner eine N,N-Dimethylamino-, N,N-Diethylamino-, N-Methylamino-, N-Ethylamino-, N,N-Methylethylamino-, N,N-Dipropylamino-, N,N-Dibutylamino-, N-Morpholino-, eine Acetoxy-, Propionyloxy- und Butyryloxygruppe und/oder eine Phenylgruppe ist, die ebenfalls substituiert sein kann.

Ganz besonders bevorzugte Ausgangsverbindungen sind solche der Formel (X) 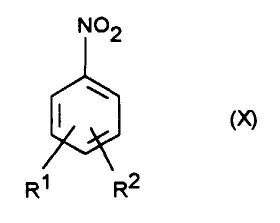 wobei R¹ und R² gleich oder verschieden sein können und Wasserstoff, C₁-C₄-Alkyl, C₁-C₄-Alkoxy, Phenyl, C₁-C₄-Alkylphenyl, Nitro, Amino, Cyano, -COOR³, -SO₂OR³ odr Sulfamoyl und R³ Wasserstoff oder das Äquivalent eines Kations bedeuten.

Besonders bevorzugte Produkte des erfindungsgemäßen Verfahrens sind demgemäß Aminoverbindungen der Formel (XI), 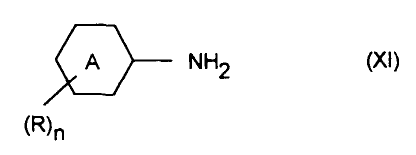 wobei die Symbole und Indizes die in der Formel (IX) angegebenen Bedeutungen haben.

Die erfindungsgemäß hergestellten Verbindungen finden vielfältige Verwendung, beispielweise als Zwischenprodukte zur Herstellung von Wirkstoffen, Polymeren und Farbstoffen.

Bevorzugt dienen die erfindungsgemäß hergestellten Verbindungen als Ausgangsstoffe zur Herstellung von Diazoniumsalzen und Benzoxazolen, wie Fenoxaprop-P-ethyl, einem selektiven Herbizid, welches unter dem Namen Puma® von der Firma AgrEvo, Frankfurt, Deutschland vertrieben wird.

Diazoniumsalze dienen beispielsweise als Zwischenprodukte zur Herstellung von Wirkstoffen, Polymeren und Farbstoffen und insbesondere als Diazotypievorprodukte.

Auf die in der Beschreibung zitierten Dokumente, die beispielsweise das technische Gebiet der Erfindung illustrieren oder die Herstellung erfindungsgemäß eingesetzter Verbindungen beschreiben, wird ausdrücklich Bezug genommen; sie gelten durch Zitat als Bestandteil dieser Anmeldung.

Auf die Offenbarung der deutschen Patentanmeldungen 195 298 74.8 und 196 193 59.1, deren Priorität die vorliegende Anmeldung beansprucht, sowie auf die Zusammenfassung dieser Anmeldung wird hiermit ausdrücklich Bezug genommen, sie gelten durch Zitat als Bestandteil dieser Anmeldung.

Die Erfindung wird durch die Beispiele näher erläutert, ohne sie dadurch beschränken zu wollen.

### Beispiel 1

### Herstellung des Katalysators Rh(CO)Cl(TPPTS)

RhCl₃ x H₂O (0,26 g, 1 mmol) wurde zu einer siedenden Lösung von TPPTS (29,2 %ig, 19,5 g, 10 mmol) gegeben. Nach ca. 15 - 20 Sekunden wurde eine Formaldehydlösung (35 %ig, 15 g, 175 mmol) zugesetzt. Die Lösung verfärbte sich von dunkelrot nach gelb. Anschließend wurde 10 min auf ca. 80°C erhitzt und solange tropfenweise mit Ethanol versetzt, bis eine leichte Trübung auftrat. Beim Abkühlen kristallisierte TPPTS sowie der Rh-TPPTS-Komplex aus der Lösung aus. Die Kristalle des Komplexes wurden durch Waschen mit Ethanol/H₂O 80/20 weitgehend von überschüssigem TPPTS befreit. Nach Umkristallisation aus Ethanol/H₂O und Trocknen im Vakuum lag der Komplex stark angereichert vor.

### Beispiel 2

### Reduktion von Nitrobenzol

In einem 2 I-Autoklaven mit Begasungsrührer wurde ein Zweiphasensystem, bestehend aus 200 g (1,62 mol) Nitrobenzol, 200 g Wasser und 0,31 g (0,235 mmol) Rh(CO)Cl(TPPTS)₂, bei 20 bar und 100°C hydriert. Nach 8 h erhielt man bei einem Umsatz von 99,3 % einen Gehalt von 87,1 % an Anilin. Die wäßrige Katalysatorlösung wurde im Scheidetrichter abgetrennt.

### Beispiel 3

### Reduktion von Nitrobenzol mit rückgeführtem Katalysator

200 g Nitrobenzol wurde unter den in Beispiel 2 angegebenen Reaktionsbedingungen in Gegenwart der aus diesem Beispiel wiedergewonnenen Katalysatorlösung reduziert. Diesmal erhielt man nach 3,5 h einen Umsatz von 98 %. Der Gehalt an Anilin betrug 95,5 %.

### Beispiel 4

### Reduktion von 2-Chlornitrobenzol mit rückgeführtem Katalysator

Unter gleichen Reaktionsbedingungen wie in Beispiel 2 wurden 200 g (1,27 mol) 2-Chlornitrobenzol mit dem aus Beispiel 2 wiedergewonnenen Katalysator umgesetzt. Nach 8,5 h erhielt man eine Lösung folgender Zusammensetzung: 11,0 % Anilin (durch reduktive Dehalogenierung), 81,8 % 2-Chloranilin sowie 3,2 % nicht umgesetztes 2-Chlornitrobenzol.

### Beispiel 5

### Reduktion von 4-Ethoxynitrobenzol

Unter gleichen Reaktionsbedingungen wie in Beispiel 2 wurden 200 g (1,31 mol) 4-Ethoxynitrobenzol in 400 g Toluol mit frischem Rhodium-Katalysator aus Beispiel 1 (0,23 mol in 200 ml Wasser) umgesetzt. Nach einer Stunde war die Reaktion beendet. Laut GC enthielt die Lösung nach der Phasentrennung 95,5 % 4-Ethoxyanilin und 1,4 % 4-Ethoxynitrobenzol.

### Beispiel 6

### Reduktion von 3-Nitrotoluol mit rückgeführtem Katalysator

Mit der aus Beispiel 5 wiedergewonnenen wäßrigen Katalysatorlösung wurden 200 g (1,87 mol) 3-Nitrotoluol in 400 g Toluol unter gleichen Reaktionsbedingungen reduziert. Nach 3 h 40 min war die Verbindung vollständig zu 3-Methylanilin umgesetzt. Edukt war in der Lösung nicht mehr nachweisbar.

### Beispiel 7

### Reduktion von 2,4-Dimethylnitrobenzol mit rückgeführtem Katalysator

Ein Gemisch aus 200 g (0,76 mol) 2,4-Dimethylnitrobenzol und zurückgeführter Katalysatorlösung wurde 10 h bei 20 bar Wasserstoffdruck auf 100°C erhitzt. Eine GC-Analyse der Produktlösung ergab einen Gehalt von 97,7 % 3,4-Dimethylanilin.

### Beispiel 8

### Reduktion von 5-Chlor-2-nitro-phenol

In einem 2l Autoklaven wurden 100 g (0,576 mol) 5-Chlor-2-nitro-phenol in 200 ml Wasser suspendiert und bei 100°C/20 bar Wasserstoff hydriert. Zu Beginn der Reaktion gab man eine Katalysatorlösung, bestehend aus 0,129 h (0,576 mmol) Pd(ac)₂, 2 ml DMSO und 3,9 ml TPPTS/H₂O-Lösung (0,6 molar) zu. Nach 7 h betrug der Umsatz 99,3 % und die Lösung hatte einen Gehalt an 5-Chlor-2-amino-phenol von 95,53 %. Die Katalysatorphase wurde durch Filtration des Produktes abgetrennt und wiederverwendet.

### Beispiel 9

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff hydriert. Zu Beginn der Reaktion gab man die Katalysatorlösung aus Beispiel 8 zu. Nach 10 h betrug der Umsatz 99,8 % und die Lösung hatte einen Gehalt von 96,5 % an 5-Chlor-2-amino-phenol. Die Katalysatorphase wurde durch Filtration des Produktes abgetrennt und wiederverwendet.

### Beispiel 10

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff hydriert. Zu Beginn der Reaktion gab man die Katalysatorlösung aus Beispiel 9 zu. Nach 5,5 h betrug der Umsatz 99,9 % und die Lösung hatte einen Gehalt von 95,1 % an 5-Chlor-2-amino-phenol. Die Katalysatorphase wurde durch Filtration des Produktes abgetrennt und wiederverwendet.

### Beispiel 11

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff hydriert. Zu Beginn der Reaktion gab man die Katalysatorlösung aus Beispiel 10 zu. Nach 9,5 h betrug der Umsatz 99,9 % und die Lösung hatte einen Gehalt von 95,6 % an 5-Chlor-2-amino-phenol. Die Katalysatorphase wurde durch Filtration des Produktes abgetrennt.

### Beispiel 12

### Reduktion von 5-Chlor-2-nitro-phenol

In einem 2l-Autoklaven wurden 100 g (0,576 mol) 5-Chlor-2-nitro-phenol in 200 ml Wasser suspendiert und bei 100°C/20 bar Wasserstoff bei pH 7,5 hydriert. Zu Beginn der Reaktion gab man eine Katalysatorlösung, bestehend aus 0,129 g (0,576 mmol) Pd(ac)₂, 2 ml DMSO und 3,9 ml TPPTS/H₂O-Lösung (0,6 molar) zu. Nach 2,5 h betrug der Umsatz 99,8 % und und die Lösung hatte einen Gehalt von 82,4 % an 5-Chlor-2-amino-phenol. Die Katalysatorphase wurde durch Filtration des Produktes abgetrennt und wiederverwendet.

### Beispiel 13

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff bei pH 7,5 hydriert. Zu Beginn der Reaktion gibt man die Katalysatorlösung aus Beispiel 12 zu. Nach 6,5 h betrug der Umsatz 99,6 % und die Lösung hatte einen Gehalt von 92,8 % an 5-Chlor-2-aminophenol. Die Katalysatorphase wurde durch Filtration des Produktes abgetrennt.

### Beispiel 14

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff bei pH 7,5 hydri rt. Zu Beginn der Reaktion gab man die Katalysatorlösung aus Beispiel 13 zu. Nach 8,5 h betrug der Umsatz 99,6 % und die Lösung hatte einen Gehalt von 95,2 % an 5-Chlor-2-amino-phenol. Die Katalysatorphase wurde durch Filtration des Produktes abgetrennt.

### Beispiel 15

### Reduktion von 5-Chlor-2-nitro-phenol mit rückgeführtem Katalysator

In einem 2l-Autoklaven wurden 100 g (0,576 mol) 5-Chlor-2-nitro-phenol bei 100°C/20 bar Wasserstoff bei pH 7,5 hydriert. Zu Beginn der Reaktion gab man die Katalysatorlösung aus Beispiel 14 zu. nach 9,5 h betrug der Umsatz 99,9 % und die Lösung hatte einen Gehalt von 94,3 % an 5-Chlor-2-amino-phenol. Die Katalysatorphase wurde durch Filtration des Produktes abgetrennt.

### Beispiel 16

### Reduktion von 5-Chlor-2-nitro-phenol

In einem 2l-Autoklaven wurden 100 g (0,576 mol) 5-Chlor-2-nitro-phenol in 200 ml Wasser suspendiert und bei 100°C/20 bar Wasserstoff hydriert. Zu Beginn der Reaktion gab man eine Katalysatorlösung bestehend aus (0,576 mmol) Ru(TPPTS)₃Cl₂ zu. Nach 2,5 h betrug der Umsatz 99,8 % und die Lösung hatte einen Gehalt von 92,4 % an 5-Chlor-2-amino-phenol. Die Katalysatorphase wurde durch Filtration des Produktes abgetrennt und wiederverwendet.

## Patentansprüche

1. Verfahren zur Reduktion von Nitrogruppen zu Aminogruppen, dadurch gekennzeichnet, daß man eine Nitroverbindung mit Wasserstoff in Gegenwart eines wasserlöslichen Metallkatalysators der allgemeinen Formel (I) umsetzt,
M(L)ₙ(Y)ₘ (I)
wobei die Symbole und Indizien folgende Bedeutungen haben:
M ist Ruthenium, Rhodium, Nickel oder Palladium;
L ist gleich oder verschieden ein wasserlöslicher Ligand;
Y ist gleich oder verschieden ein weiterer Ligand oder ein Alkali- oder Erdalkaliion;
n ist 1, 2, 3 oder 4;
m ist 0, 1 oder 2.

2. Verfahren nach Anspruch 1, dadurch gekennzeichnet, daß der wasserlösliche Ligand mindestens einen Substituenten aus der folgenden Gruppe enthält: Sulfonsäuren, Sulfonsäuresalze, Carbonsäuren, Carbonsäuresalze, Phosphonsäuren, Phosphonsäuresalze, Phosphoniumsalze, Phosphinsäuren, Phosphinsäuresalze, Peralkylammoniumgruppen, Hydroxygruppen und Polyethergruppen.

3. Verfahren nach Anspruch 2, dadurch gekennzeichnet, daß man den Liganden aus der Gruppe Phosphane, Phosphite, Phosphinigsäureester, Phosphonigsäureester, Phosphole, Bipyridine, Phenanthroline, Porphyrine und Alizarine wählt.

4. Verfahren nach Anspruch 3, dadurch gekennzeichnet, daß man als wasserlöslichen Liganden ein wasserlösliches Triarylphosphan, das neben den die Wasserlöslichkeit bewirkenden Substituenten weitere Substituenten enthalten kann, oder ein wasserlösliches Phosphan der allgemeinen Formel (II) bis (VIII) einsetzt: 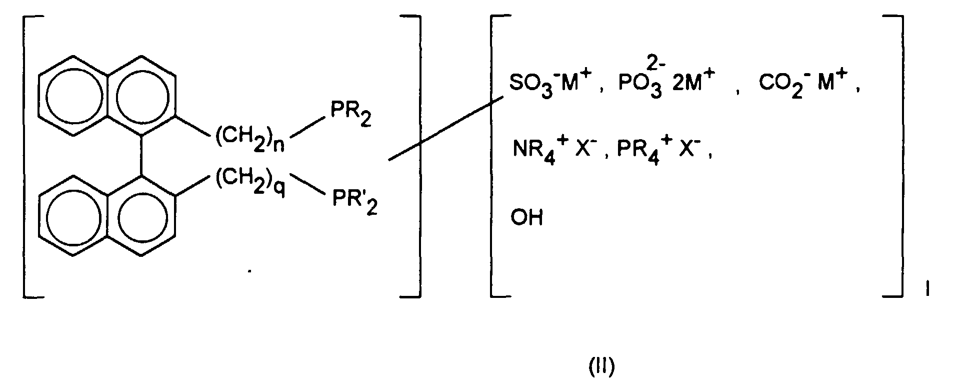 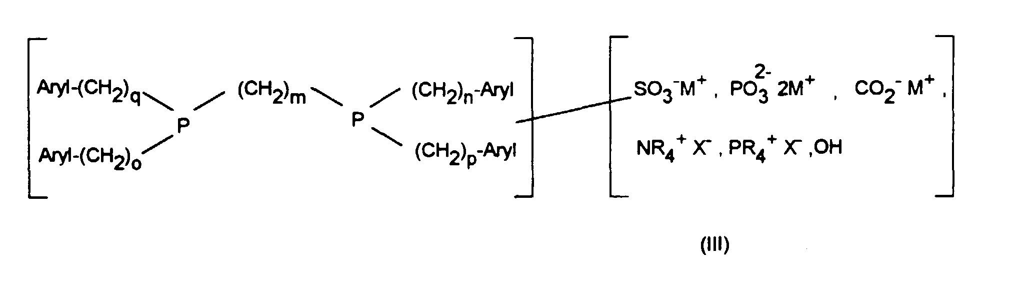 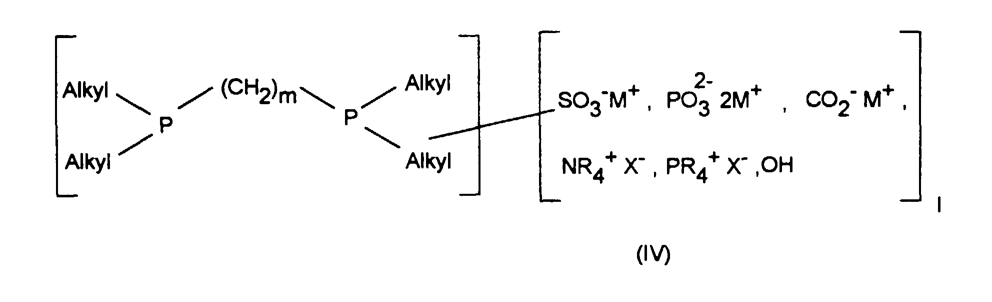 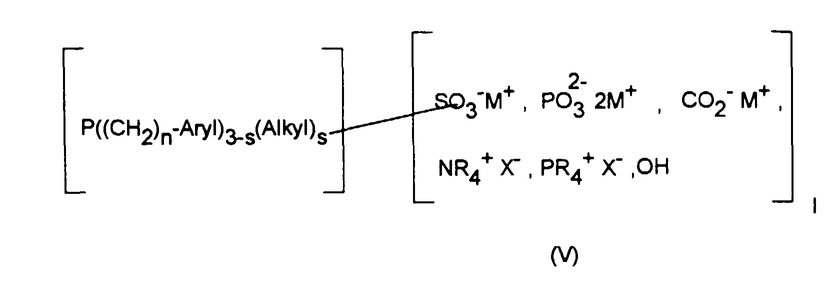 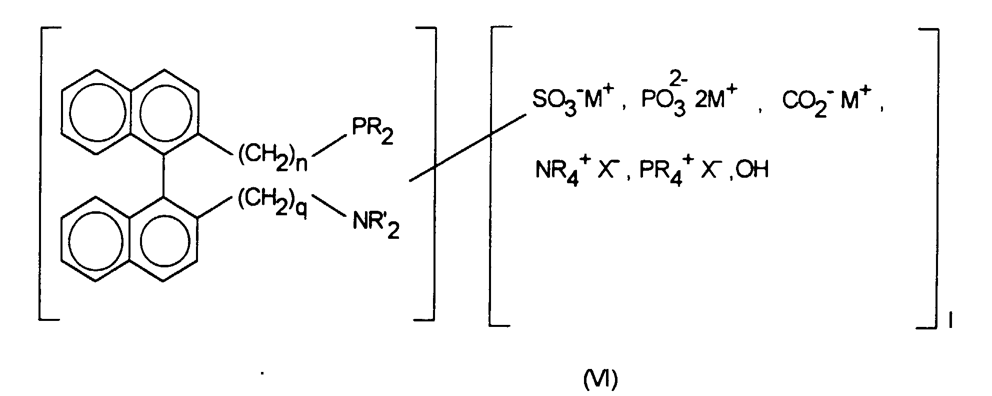 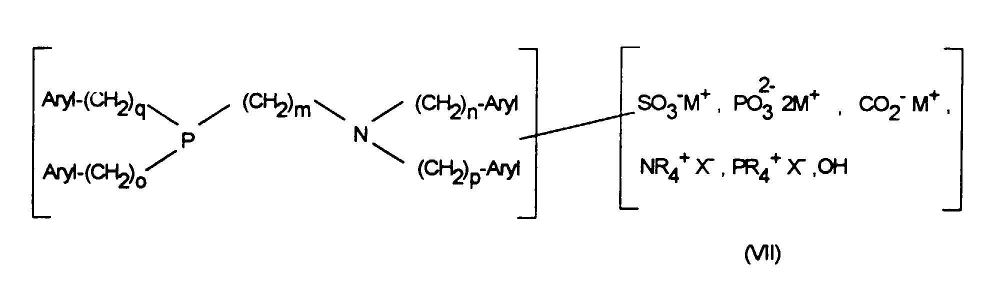 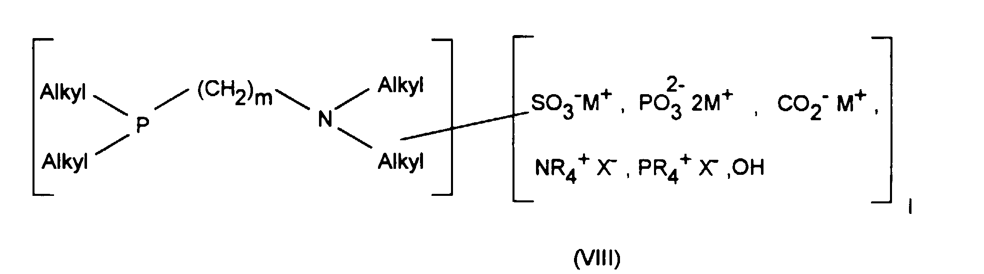 wobei die Symbole und Indizes folgende Bedeutungen haben:
Aryl: eine Phenyl- oder Naphthylgruppe, die auch einen oder mehrere Substituenten R tragen kann;
Alkyl: : eine geradkettige oder verzweigte Alkylgruppe mit 1 bis 8 Kohlenstoffatomen;
R, R': Alkyl, Aryl oder Aralkyl mit 1 bis 18 Kohlenstoffatomen;
M: Alkali-, Erdalkalimetall oder NR₄;
X: Halogen, BF₄, PF₆, OSO₂CF₃, 1/2[SO₄];
l,m: 1 bis 8;
n,o,p,q: 0, 1 bis 8;
s: 0, 1 bis 3.

5. Verfahren nach einem oder mehreren der vorhergehenden Ansprüche, dadurch gekennzeichnet, daß man ein Verhältnis Katalysator: Substrat von 1:100 bis 1:100 000 wählt.

6. Verfahren nach einem oder mehreren der vorhergehenden Ansprüche, dadurch gekennzeichnet, daß man bei einer Reaktionstemperatur von 20 bis 150°C arbeitet.

7. Verfahren nach einem oder mehreren der vorhergehenden Ansprüche, dadurch gekennzeichnet, daß man bei ainem Wasserstoffdruck von 1 bis 150 bar arbeitet.

8. Verfahren nach einem oder mehreren der vorhergehenden Ansprüche, dadurch gekennzeichnet, daß man die Nitroverbindung mit einem organischen Lösungsmittel aus der Gruppe Toluol, o-Xylol, m-Xylol, p-Xylol, Gemische isomerer Xylole, Ethylbenzol, Mesitylen, Chlorbenzol, Dichlorbenzol, Chlortoluol, Cyclohexan, Cumol, Dekalin, Ethylacetat, Butylacetat oder einem Gemisch aus zwei oder mehreren dieser Lösungsmittel verdünnt.

9. Verwendung einer Aminoverbindung, hergestellt mit einem Verfahren nach einem oder mehreren der Ansprüche 1 bis 8, als Zwischenstufe zur Herstellung von Wirkstoffen, Polymeren und/oder Farbstoffen.

10. Verfahren zur Herstellung von Rh(CO)Cl(TPPTS)₂, dadurch gekennzeichnet, daß man eine wäßrige Lösung von TPPTS und Rhodiumtrichlorid mit Formaldehyd versetzt und anschließend erhitzt.

## Claims

1. A process for reducing nitro groups to amino groups, which comprises reacting a nitro compound with hydrogen in the presence of a water-soluble metal catalyst of the formula (I),
M(L)ₙ(Y)ₘ (I)
where the symbols and indices have the following meanings:
M is ruthenium, rhodium, nickel or palladium;
L are identical or different and are each a water-soluble ligand;
Y are identical or different and are each a further ligand or an alkali metal ion or an alkaline earth metal ion;
n is 1, 2, 3 or 4;
m is 0, 1 or 2.

2. The process as claimed in claim 1, wherein the water-soluble ligand contains at least one substituent selected from the following group: sulfonic acids, sulfonate salts, carboxylic acids, carboxylate salts, phosphonic acids, phosphonate salts, phosphonium salts, phosphinic acids, phosphinate salts, peralkylammonium groups, hydroxy groups and polyether groups.

3. The process as claimed in claim 2, wherein the ligand is selected from the group consisting of phosphines, phosphites, phosphinous esters, phosphonous esters, phospholes, bipyridines, phenanthrolines, porphyrins and alizarins.

4. The process as claimed in claim 3, wherein the water-soluble ligand is a water-soluble triarylphosphine which can contain, apart from the substituents which make it water-soluble, further substituents, or a water-soluble phosphine of the formulae (II) to (VIII): 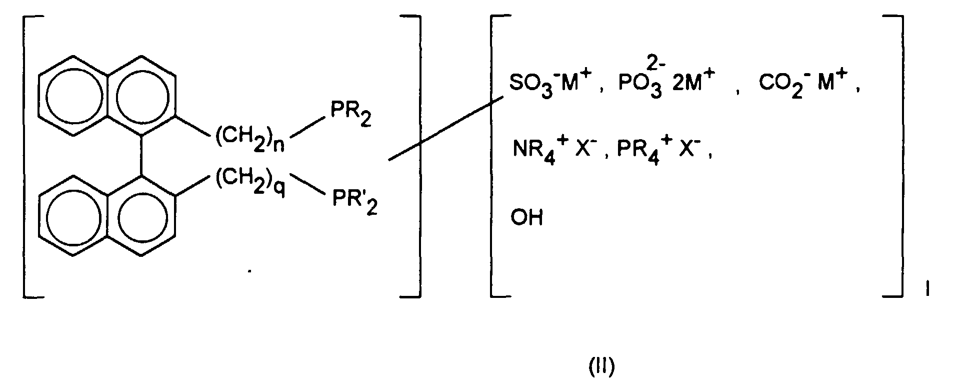 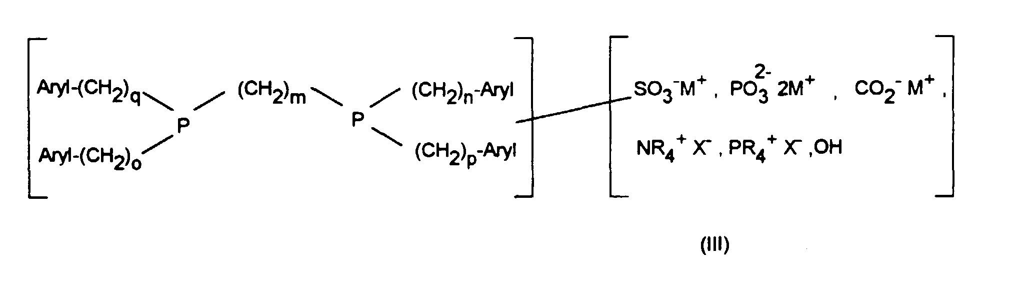 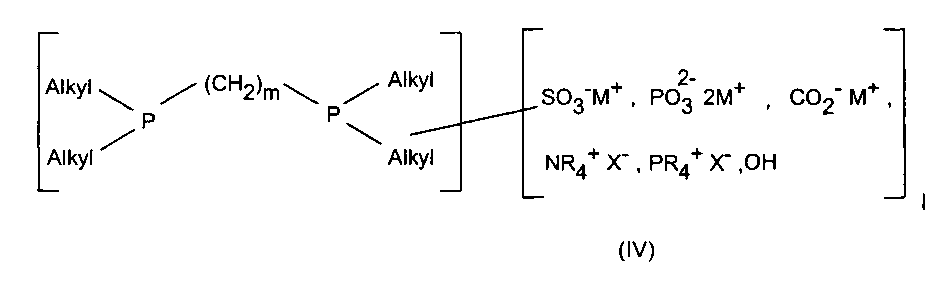 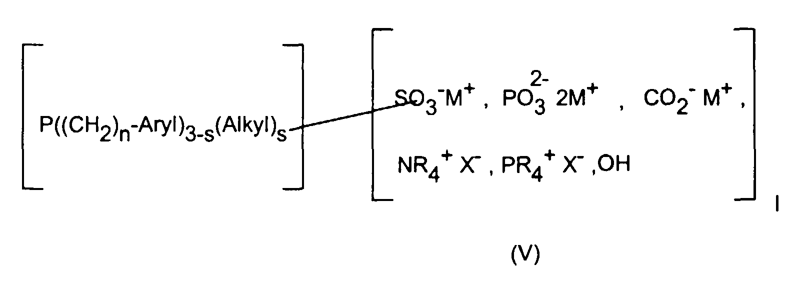 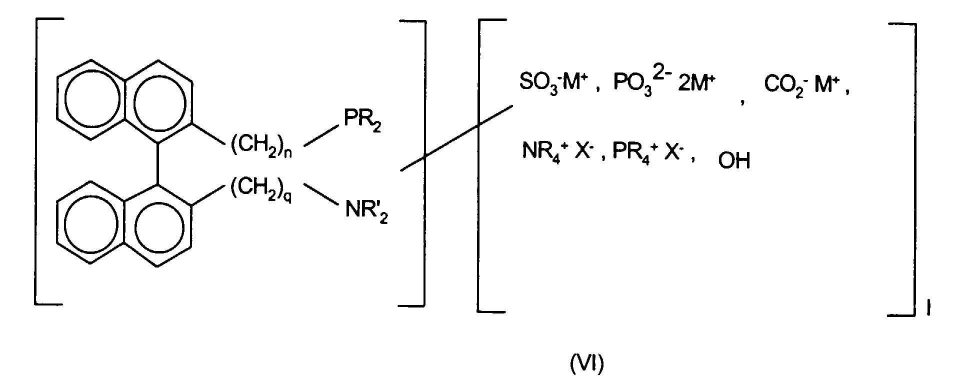 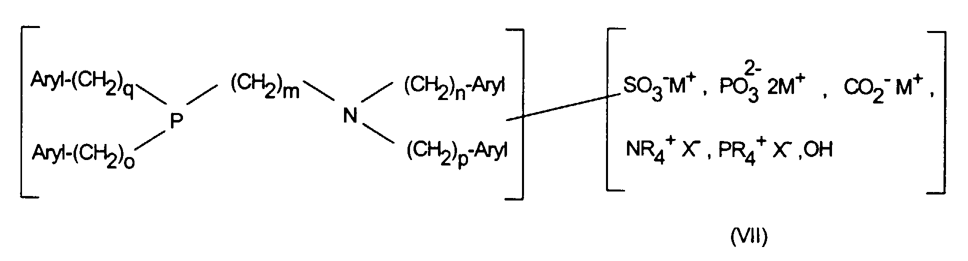 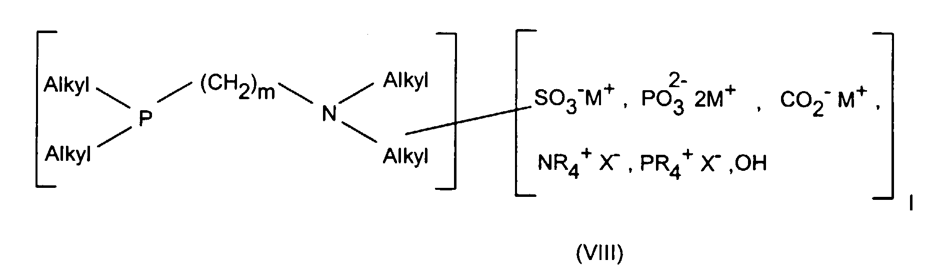 where the symbols and indices have the following meanings:
Aryl: a phenyl or naphthyl group which may also bear one or more substituents R;
Alkyl: a straight-chain or branched alkyl group having from 1 to 8 carbon atoms;
R, R': alkyl, aryl or aralkyl having from 1 to 18 carbon atoms;
M: alkali metal, alkaline earth metal or NR₄;
X: halogen, BF₄, PF₆, OSO₂CF₃, 1/2[SO₄];
I, m: 1 to 8;
n, o, p, q: 0, 1 to 8;
S: 0, 1 to 3.

5. The process as claimed in one or more of the preceding claims, wherein a ratio of catalyst:substrate of from 1:100 to 1:100,000 is selected.

6. The process as claimed in one or more of the preceding claims, wherein the reaction temperature employed is from 20 to 150°C.

7. The process as claimed in one or more of the preceding claims, wherein the hydrogen pressure employed is from 1 to 150 bar.

8. The process as claimed in one or more of the preceding claims, wherein the nitro compound is diluted with an organic solvent selected from the group consisting of toluene, o-xylene, m-xylene, p-xylene, mixtures of isomeric xylenes, ethylbenzene, mesitylene, chlorobenzene, dichlorobenzene, chlorotoluene, cyclohexane, cumene, decalin, ethyl acetate, butyl acetate or a mixture of two or more of these solvents.

9. The use of an amino compound prepared using a process as claimed in one or more of claims 1 to 8 as intermediate for preparing active compounds, polymers and/or dyes.

10. A process for preparing Rh(CO)Cl(TPPTS)₂, which comprises admixing an aqueous solution of TPPTS and rhodium trichloride with formaldehyde and subsequently heating it.

## Revendications

1. Procédé de réduction de groupes nitro en groupes amino, caractérisé en ce que l'on fait réagir un composé nitro avec de l'hydrogène en présence d'un catalyseur métallique soluble dans l'eau, dont la formule générale (I) est
M(L)ₙ(Y)ₘ (I)
les symboles et indices ayant les significations suivantes :
M est du ruthénium, du rhodium, du nickel ou du palladium;
L est un ligand, identique ou différent, soluble dans l'eau;
Y est un autre ligand, identique ou différent, ou un ion alcalin ou alcalino-terreux,
n est 1, 2, 3 ou 4 ;
m est 0, 1 ou 2.

2. Procédé selon la revendication 1, caractérisé en ce que le ligand soluble dans l'eau contient au moins un substituant choisi dans le groupe consitué des acides sulfoniques, sels d'acides sulfoniques, acides carboxyliques, sels d'acides carboxyliques, acides phosphoniques, sels d'acides phosphoniques, sels de phosphonium, acides phosphiniques, sels d'acides phosphiniques, groupes peralkylammonium, groupes hydroxyles et groupes polyéthers.

3. Procédé selon la revendication 2, caractérisé en ce que le ligand est choisi dans le groupe des phosphanes, phosphites, esters d'acides phosphiniques, esters d'acides phosphoniques, phospholes, bipyridines, phénanthrolines, porphyrines et alizarines.

4. Procédé selon la revendication 3, caractérisé en ce que l'on utilise comme ligands solubles dans l'eau un triarylphosphane soluble dans l'eau qui, outre les substituants provoquant la solubilité dans l'eau, peut contenir d'autres substituants, ou un phosphane soluble dans l'eau de formule générale (II) à (VIII) : 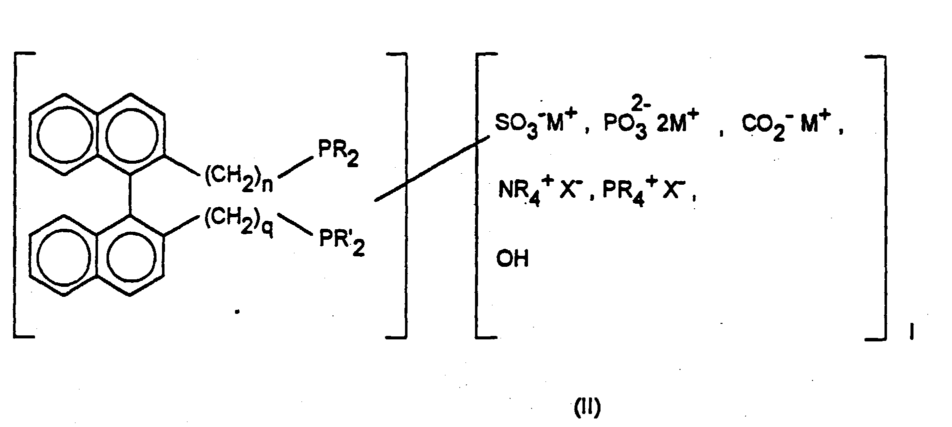 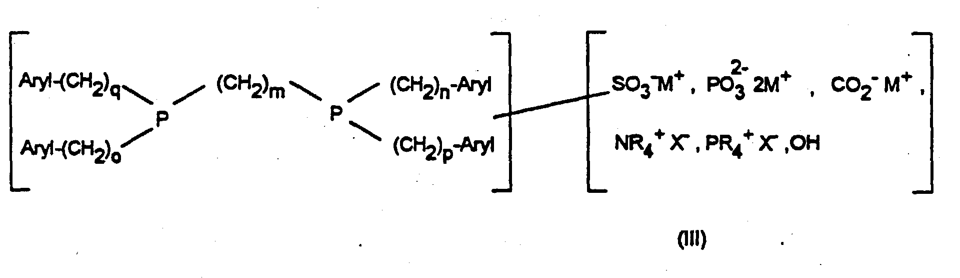 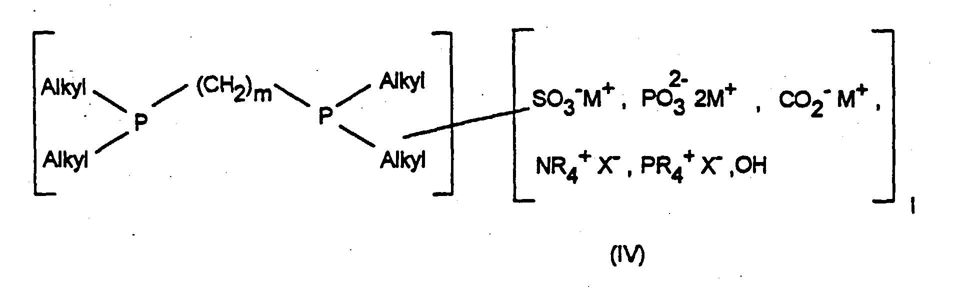 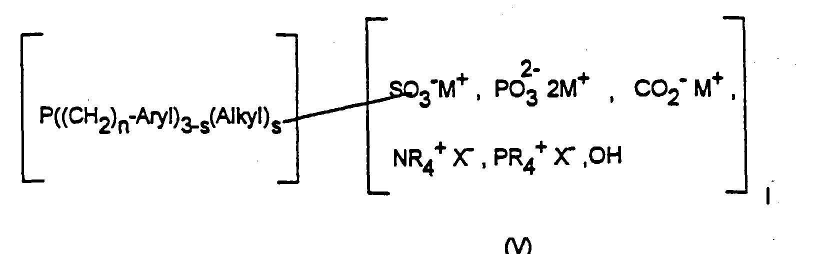 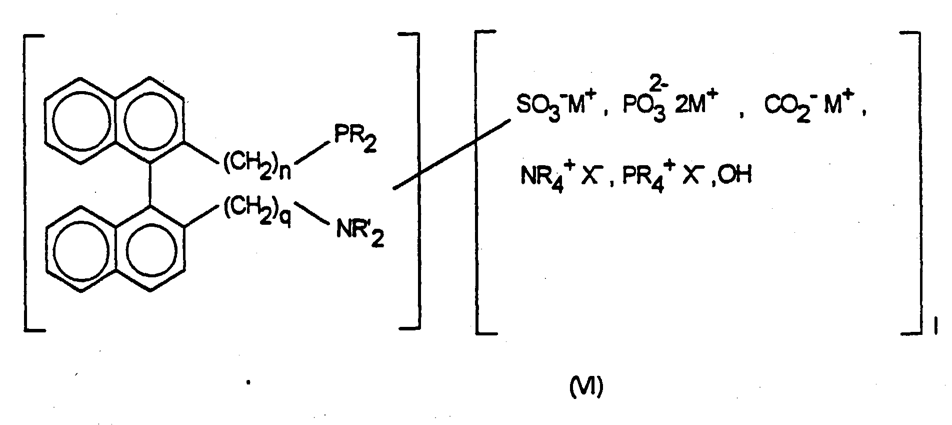 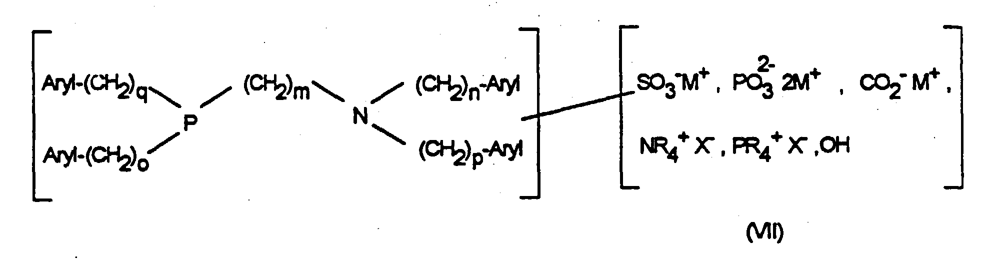 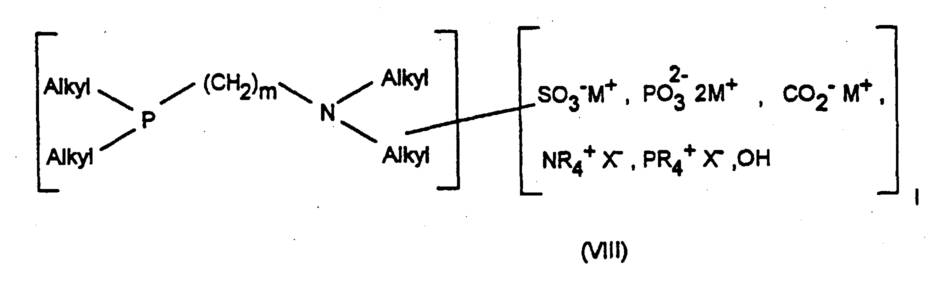 les symboles et indices ayant les significations suivantes :
Aryl : un groupe phényle ou naphtyle qui peut porter également un ou plusieurs substituants R ;
Alkyl : un groupe alkyle linéaire ou ramifié avec de 1 à 8 atomes de carbone ;
R,R' : alkyle, aryle ou aralkyle avec de 1 à 18 atomes de carbone ;
M : métal alcalin ou alcalino-terreux ou NR₄ ;
X : halogène, BF₄, PF₆, OSO₂CF₃, 1/2[SO₄] ;
l,m : 1 à 8 ;
n,o,p,q : 0, 1 à 8 ;
s : 0, 1 à 3.

5. Procédé selon l'une ou plusieurs des revendications qui précèdent, caractérisé en ce que le rapport catalyseur : substrat est de 1:100 à 1:100 000.

6. Procédé selon l'une ou plusieurs des revendications qui précèdent, caractérisé en ce que la température de réaction est de 20 et 150°C.

7. Procédé selon l'une ou plusieurs des revendications qui précèdent, caractérisé en ce que la pression d'hydrogène est de 1 à 150 bar.

8. Procédé selon l'une ou plusieurs des revendications qui précèdent, caractérisé en ce que l'on dilue le composé nitro avec un solvant organique du groupe constitué du toluène, de l'o-xylène, du m-xylène, du p-xylène, des mélanges d'isomères des xylènes, de l'éthylbenzène, du mésitylène, du chlorobenzène, du dichlorobenzène, du chlorotoluène, du cyclohexane, du cumène, de la décaline, de l'acétate d'éthyle, de l'acétate de butyle ou avec un mélange de deux ou de plusieurs de ces solvants.

9. Utilisation d'un composé aminé préparé suivant un procédé selon l'une ou plusieurs des revendications 1 à 8 en tant qu'étape intermédiaire de la préparation de substances actives, de polymères et/ou de colorants.

10. Procédé pour la préparation de Rh(CO)Cl(TPPTS)₂, caractérisé en ce que l'on ajoute à une solution aqueuse de TPPTS et de trichlorure de rhodium du formaldéhyde et on la chauffe ensuite.
